(19) Europäisches Patentamt
European Patent Office
Office européen des brevets

(11) EP 1 944 062 A2

(12) DEMANDE DE BREVET EUROPEEN

(43) Date de publication:
**16.07.2008 Bulletin 2008/29**

(51) Int Cl.:
***A61Q 5/06** (2006.01)* ***A61K 8/87** (2006.01)*

(21) Numéro de dépôt: **08300016.6**

(22) Date de dépôt: **09.01.2008**

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(30) Priorité: **12.01.2007 FR 0752652**

(71) Demandeur: **L'Oréal**
**75008 Paris (FR)**

(72) Inventeurs:
- **Rollat-Corvol, Isabelle**
  **75017, PARIS (FR)**
- **Benabdillach, Katarina**
  **95130, LE PLESSIS-BOUCHARD (FR)**

(74) Mandataire: **Catherine, Alain et al**
**Cabinet Harlé & Phélip**
**7, rue de Madrid**
**75008 Paris (FR)**

# (54) Composition cosmétique comprenant un polyuréthane cationique, un tensiooactif, et un corps gras et application au coiffage

(57) Composition cosmétique comprenant, dans un milieu aqueux cosmétiquement acceptable :
(i) au moins un polyuréthane cationique comportant au moins un motif non ionique dérivé d'un homopolymère ou d'un copolymères d'oléfine,
(ii) au moins un tensioactif choisi parmi un tensioactif anionique, non ionique ou cationique, amphotère, et leurs mélanges, et
(iii) au moins un corps gras.

EP 1 944 062 A2

## Description

La présente invention concerne de nouvelles compositions cosmétiques, notamment de coiffage, comprenant l'association d'au moins un polyuréthane cationique comportant des motifs non ioniques dérivés d'au moins un homo- ou copolymère d'oléfine, au moins un tensioactif et au moins un corps gras.

L'utilisation des polyuréthanes cationiques à caractère élastique dans des compositions cosmétiques, notamment des compositions de coiffage, est connue.

Ainsi la demande de brevet français FR 2 815 350 décrit des polyuréthanes cationiques à caractère élastique et leur utilisation pour la formulation de laques et de compositions de coiffage améliorant la souplesse de la coiffure, c'est-à-dire permettant d'obtenir un maintien des cheveux plus naturel que celui obtenue avec des polymères fixants usuels.

La demande de brevet français FR-2 833 960 décrit des compositions cosmétiques de coiffage, et en particulier des compositions de coiffage à rincer, notamment des shampoings coiffants, comportant un polyuréthane cationique ou amphotère auto-adhésif.

Cependant, les propriétés de tenue de la mise en forme obtenues ne sont pas totalement satisfaisantes.

De manière surprenante, la demanderesse a trouvé qu'en associant à ces polyuréthanes cationiques comportant des motifs dérivés d'un homo-et/ou copolymère d'oléfine à un tensioactif et à un corps gras, il était possible de formuler des compositions cosmétiques de coiffage présentant un excellent contrôle de la tenue et du volume de la chevelure, dans le temps.

L'invention a par conséquent pour objet une composition cosmétique, et en particulier une composition de coiffage, contenant, dans un milieu cosmétiquement acceptable, au moins un polyuréthane cationique comportant au moins un motif dérivé d'un homo- et/ou copolymère d'oléfine, au moins un tensioactif et au moins un corps gras.

L'invention a également pour objet une telle composition sous forme d'émulsion.

L'invention a encore pour objet un procédé de coiffage comprenant l'application sur les cheveux d'une telle composition, un rinçage éventuel des cheveux, puis la mise en forme et le séchage des cheveux.

Selon l'invention, la composition cosmétique comprend, dans un milieu aqueux cosmétiquement acceptable :

(i) au moins un polyuréthane cationique comportant au moins un motif non ionique dérivé d'un homo- et/ou copolymère d'oléfine, et
(ii) au moins un agent tensioactif, et
(iii) au moins un corps gras.

Le premier constituant essentiel des compositions de l'invention est le polyuréthane cationique comportant au moins un motif non ionique, dérivé d'un homo-ou copolymère d'oléfine.

Les polyuréthanes cationiques convenant de préférence pour l'invention contiennent :

(a) des motifs cationiques dérivés d'au moins un composé, de préférence une amine tertiaire ou quaternaire, présentant au moins deux fonctions réactives à hydrogène labile,
(b) des motifs non ioniques dérivés de polymères non ioniques portant à leurs extrémités des fonctions réactives à hydrogène labile, au moins un des motifs (b), de préférence au moins 50% en poids des motifs (b) et mieux la totalité des motifs (b) étant un motif ou des motifs (b1) dérivant d'un homo-ou copolymère d'oléfine portant à ses extrémités des fonctions réactives à hydrogène labile, et
(c) des motifs dérivés d'au moins un diisocyanate.

Par motif cationique, on entend au sens de la présente invention tout motif qui, soit par sa nature chimique propre, soit en fonction du milieu et/ou du pH dans lequel il se trouve, sera sous forme cationique.

On entend par fonctions réactives à hydrogène labile des fonctions capables, après départ d'un atome d'hydrogène, de former des liaisons covalentes avec les fonctions isocyanate des composés formant les motifs (b). On peut citer à titre d'exemple de telles fonctions les groupes hydroxyle, amine primaire ($-NH_2$) ou amine secondaire (-NHR), ou encore les groupes thiol (-SH).

La polycondensation de composés portant ces fonctions réactives à hydrogène labile avec des diisocayanates donne, selon la nature des fonctions réactives portant l'hydrogène labile (-OH, $-NH_2$, -NHR ou -SH), respectivement des polyuréthanes, des polyurées ou des polythio-uréthanes. Tous ces polymères sont regroupés dans la présente demande, par souci de simplification, sous le terme de polyuréthanes. De préférence les polymères de l'invention sont de vrais polyuréthanes.

Lorsque les amines tertiaires ou quaternaires formant les motifs (a) portent plus de deux fonctions à hydrogène labile les polyuréthanes obtenus présentent une structure ramifiée.

Dans un mode de réalisation préféré des polyuréthanes de la présente invention, les amines tertiaires ou

quaternaires formant les motifs cationiques

(a) ne présentent que deux fonctions réactives à hydrogène labile et les polyuréthanes obtenus par polycondensation ont par conséquent une structure essentiellement linéaire.

Il est bien entendu également possible d'utiliser un mélange d'amines difonctionnelles contenant une faible proportion d'amines portant plus de deux fonctions réactives à hydrogène labile.

Les amines tertiaires ou quaternaires formant les motifs cationiques (a) sont de préférence choisies parmi les composés correspondant à l'une des formules suivantes :

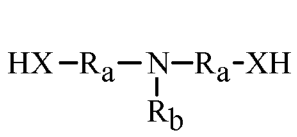

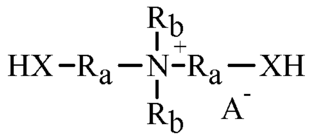

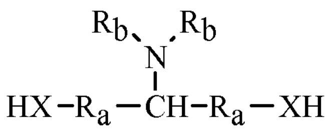

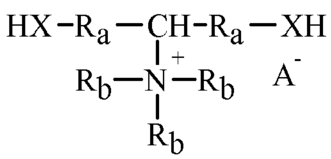

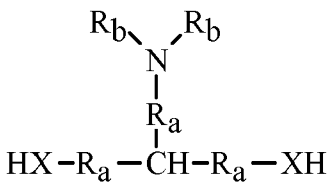

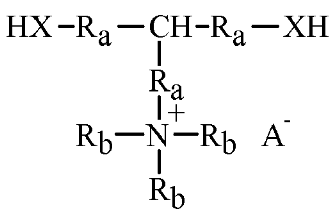

dans lesquelles

chaque $R_a$ représente indépendamment un groupe alkylène en $C_{1-6}$, linéaire ou ramifié, cycloalkylène en $C_{3-6}$ ou arylène, tous pouvant être substitués par un ou plusieurs atomes d'halogène et comporter un ou plusieurs hétéroatomes choisis parmi O, N, P et S,

chaque $R_b$ représente indépendamment un groupe alkyle en $C_{1-6}$, cycloalkyle en $C_{3-6}$ ou aryle, tous pouvant être substitués par un ou plusieurs atomes d'halogène et comporter un ou plusieurs hétéroatomes choisis parmi O, N, P et S,

chaque X représente indépendamment un atome d'oxygène ou de soufre ou un groupe NH ou $NR_c$, où $R_c$ représente un groupe alkyle en $C_{1-6}$, et

A- représente un contre-ion physiologiquement acceptable.

On peut citer à titre d'amines tertiaires particulièrement préférées pour l'obtention des polyuréthanes cationiques, la N-méthyldiéthanolamine et la N-tert-butyldiéthanolamine.

Les amines tertiaires et quaternaires formant les motifs cationiques (a) des polyuréthanes de la présente invention peuvent également être des polymères à fonction(s) amine tertiaire et/ou quaternaire, portant à leurs extrémités des fonctions réactives à hydrogène labile. La masse molaire moyenne en poids de ces polymères à fonctions amine tertiaire et/ou quaternaire est de préférence comprise entre 400 et 10 000.

On peut citer à titre d'exemples de tels polymères à fonctions amine appropriés les polyesters issus de la polycondensation de la N-méthyldiéthanolamine et de l'acide adipique.

Lorsque les amines formant les motifs cationiques (a) sont des composés à fonction(s) amine tertiaire, une partie ou la totalité de ces fonctions amine doit être neutralisée par un agent de neutralisation approprié choisi parmi les acides organiques ou minéraux physiologiquement acceptables. On peut citer à titre d'exemple d'acides préférés l'acide chlorhydrique ou l'acide acétique.

Le deuxième type de motifs formant les polyuréthanes préférés de la présente invention sont des motifs macromoléculaires, appelés motifs (b), dérivés de polymères non ioniques portant à leurs extrémités des fonctions réactives à hydrogène labile et ayant de préférence une température de transition vitreuse (Tg), mesurée par analyse anthalpique différentielle, inférieure à 10°C.

Selon l'invention, au moins un de ces motifs (b1) dérive d'un homo- ou copolymère d'oléfine.

Les propriétés viscoélastiques des polyuréthanes sont particulièrement avantageuses lorsque les motifs (b) sont dérivés de polymères ayant une température de transition vitreuse inférieure à 0°C et mieux encore inférieure à -10°C.

Ces polymères ont de préférence une masse molaire moyenne en poids comprise entre 400 et 10 000 et plus particulièrement entre 1000 et 5000.

Les polymères non ioniques susceptibles de former les motifs non ioniques (b2) différents des motifs non-ioniques (b1) dérivant des homo- et copolymères d'oléfine, peuvent être choisis parmi les polyéthers, les polyesters, les polysiloxanes, les polycarbonates et les polymères fluorés.

De préférence, les polymères susceptibles de former les motifs non-ioniques (b) sont choisis uniquement parmi les homo- et copolymères d'oléfine.

Parmi les homopolymères d'oléfine terminés par des groupes réactifs à hydrogène labile convenant pour la présente invention on peut citer les homopolymères et les copolymères statistiques ou blocs d'éthylène, de propylène, 1-butylène, 2-butylène, isobutylène, 1,2-butadiène, 1,4-butadiène et d'isoprène.

Les homo- et copolymères de butadiène et d'isoprène peuvent être partiellement ou totalement hydrogénés.

Les polymères préférés sont les copolymères d'éthylène/butylène, les polybutadiènes et les polybutadiènes hydrogénés, porteur de groupes réactifs à hydrogène labile à leurs extrémités et en particulier de groupes hydroxyles. Encore plus préférentiellement ces polymères sont des 1,2 et/ou 1,4 polybutadiènes.

De tels polymères sont disponibles commercialement par exemple sous la dénomination KRATON® L, en particulier KRATON® L 2203 (polybutadiènediol hydrogéné) auprès de la société KRATON polymers, KRASOL LBH® et LBHP®, notamment KRASOL LBHP® 2000 (polybutadiène diol) auprès de la société SARTOMER et G1® 3000 (copolymère éthylène/butylène) auprès de la société NISSO CHEMICAL.

Les diisocyanates formant les motifs (c) englobent les diisocyanates aliphatiques, alicycliques ou aromatiques.

Des diisocyanates préférés sont choisis parmi le méthylènediphényldiisocyanate, le méthylènecyclohexanediisocyanate, l'isophoronediisocyanate, le toluènediisocyanate, le natphtalènediisocyanate, le butanediisocyanate et l'hexyldiisocyanate. Ces diisocyanates peuvent bien entendu être utilisés seuls ou sous forme de mélange de deux ou plusieurs diisocyanates. Encore plus préférentiellement l'isocyanate et l'isophorone diisocyanate.

Comme indiqué ci-avant, les polyuréthanes cationiques préférés de la présente invention peuvent contenir, en plus des motifs (a), (b1) et (c), une certaine fraction de motifs (b2) dérivés de composés non ioniques monomères contenant au moins deux fonctions à hydrogène labile et différents des monomères conduisant aux motifs (b1).

Ces motifs (b2) sont dérivés par exemple de néopentylglycol, d'hexaéthylèneglycol, de 1,2-éthanediol, de 1,2-propanediol et de 1,3-propanediol ou d'aminoalcools en $C_1$-$C_6$ par exemple l'aminoéthanol.

Les polyuréthanes cationiques de l'invention présentent de préférence un caractère élastique.

Un paramètre physique caractérisant les propriétés visco-élastiques des polyuréthanes cationiques ci-dessus est leur recouvrance en traction. Cette recouvrance est déterminée par essai de fluage en traction consistant à étirer rapidement une éprouvette jusqu'à un taux d'allongement prédéterminé, puis à relâcher la contrainte et à mesurer la longueur de l'éprouvette.

L'essai de fluage utilisé pour la caractérisation des polyuréthanes cationiques à caractère élastique de la présente invention se déroule de la manière suivante :

On utilise, comme éprouvette, un film du polyuréthane ayant une épaisseur de 500 $\pm$ 50 mm, découpé en bandes de 80 mm x 15 mm. Ce film de copolymère est obtenu par séchage, à une température de 22 $\pm$ 2 °C et à une humidité relative de 50 $\pm$ 5 %, d'une solution ou dispersion à 3 % en poids dudit polyuréthane dans de l'eau et/ou de l'éthanol.

Chaque bande est fixée entre deux mors, distants de 50 $\pm$ 1 mm l'un de l'autre, et est étirée à une vitesse de 20 mm/minute (dans les conditions de température et d'humidité relative ci-dessus) jusqu'à un allongement de 50 % ($\varepsilon_{max}$), c'est-à-dire jusqu'à 1,5 fois sa longueur initiale. On relâche alors la contrainte en imposant une vitesse de retour égale à la vitesse de traction, soit 20 mm/minute, et on mesure l'allongement de l'éprouvette (exprimé en % par rapport à la longueur initiale) immédiatement après retour à charge nulle ($\varepsilon_i$).

La recouvrance instantanée ($R_i$) est calculée à l'aide de la formule suivante :

$$R_i\ (\%) = ((\varepsilon_{max} - \varepsilon_i)/\ \varepsilon_{max}) \times 100$$

Les polyuréthanes cationiques à caractère élastique de la présente invention ont de préférence une recouvrance instantanée ($R_i$), mesurée dans les conditions indiquées ci-dessus, comprise entre 5 % et 95 %, en particulier comprise entre 20 % et 90 % et idéalement entre 35 et 85 %.

La température de transition vitreuse (Tg) des polymères non ioniques formant les motifs (a2) et des polyuréthanes cationiques de la présente invention est mesurée par analyse enthalpique différentielle (DSC, *differential scanning calorimetry)* selon la norme ASTM D3418-97.

Les polyuréthanes cationiques à caractère élastique de la présente invention présente de préférence au moins deux températures de transition vitreuse, dont une au moins est inférieure à 10°C, de préférence inférieure à 0°C et mieux encore inférieure à -10°C, et au moins une autre est supérieure ou égale à la température ambiante (20°C).

La recouvrance instantanée et par conséquent les propriétés viscoélastiques des polyuréthanes de la présente invention dépend des fractions des différents motifs monomères (a), (b1), (b2) et (c).

La fraction de motifs (a) doit être de préférence suffisante pour conférer aux polymères leur charge positive responsable de leur bonne affinité pour les substrats kératiniques. Les motifs (b) doivent représenter de préférence une fraction en poids suffisante pour que les polyuréthanes présentent de préférence au moins une température de transition vitreuse inférieure à 10 °C et ne forment pas des films cassants.

De manière préférée, les motifs (a) représentent de 0,1 à 90 %, de préférence de 1 à 30 %, mieux de 5 à 25% et idéalement de 5 à 10% en poids, les motifs (b1) de 10 à 99,9 %, de préférence de 20 à 99 % et mieux de 30 à 85% en poids et les motifs (b2) de 0 à 50 % en poids, de préférence de 0 à 30 % en poids total des motifs du polyuréthane. De préférence encore les polyuréthanes de l'invention ne comportent pas de motif (b2).

De manière préférée, les motifs (c) représentent de 1 à 60%, mieux de 5 à 50%, idéalement de 10 à 40% du poids total des motifs du polyuréthane.

Les motifs (c) sont présents en une quantité essentiellement stoechiométrique par rapport à la somme des motifs (a) et (b). En effet, l'obtention de polyuréthanes ayant des masses molaires importantes suppose un nombre de fonctions isocyanate pratiquement identique au nombre de fonction à hydrogène labile. L'homme du métier saura choisir un éventuel excès molaire de l'un ou de l'autre type de fonction pour ajuster la masse molaire à la valeur désirée.

Dans un mode de réalisation particulier de l'invention, le polyuréthane cationique ne comprend pas de motifs en plus des motifs (a), (b) et (c). Un polyuréthane répondant à cette définition est le polyuréthane (A) décrit dans l'exemple 1.

Dans un mode de réalisation alternatif, le polyuréthane cationique comprend des motifs en plus des motifs (a), (b) et (c). Un polyuréthane répondant à cette définition est le polyuréthane (B) décrit dans l'exemple 2.

La quantité de polyuréthane présente dans une composition cosmétique objet de l'invention, dépend bien entendu du type de composition et des propriétés recherchées et peut varier à l'intérieur d'une gamme très large, comprise généralement de préférence entre 0,01 et 40 % en poids, de préférence entre 0,05 et 20 %, idéalement entre 0,1 et 10% en poids, rapporté à la composition cosmétique finale.

Le second constituant essentiel des compositions de l'invention est un agent tensioactif. Celui-ci est choisi parmi les tensioactifs anioniques, non ioniques, cationiques, et amphotères ou les mélanges de ceux-ci.

Parmi les tensioactifs anioniques utilisables, seuls ou en mélanges, dans le cadre de la présente invention, on peut notamment citer les sels, et en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants : les alkylsulfates, les alkyléthersulfates, les alkylamidoéthersulfates, les alkylarylpolyéthersulfates, les monoglycéride-sulfates ; les alkylsulfonates, les alkylamidesulfonates, les alkyl-arylsulfonates, les $\alpha$-oléfine-sulfonates, les paraffine-sulfonates ; les alkylsulfosuccinates, les alkyléthersulfosuccinates, les alkylamide-sulfosuccinates ; les alkylsulfoacétates ; les acylsarconisates ; et les acylglutamates, les groupes alkyle et acyle de tous ces composés comportant de 6 à 24 atomes de carbone et le groupe aryle désignant de préférence un groupe phényle ou benzyle.

On peut également utiliser dans le cadre de la présente invention les esters d'alkyle en $C_6$-$C_{24}$ et d'acides polyglycoside-carboxyliques tels que les glucoside-citrates d'alkyle, les polyglycoside-tartrates d'alkyle, et les polyglycoside-sulfosuccinates d'alkyle ; les alkylsulfosuccinamates, les acyliséthionates et les N-acyltaurates, le groupe alkyle ou acyle de tous ces composés comportant de 12 à 20 atomes de carbone. Parmi les tensioactifs anioniques encore utilisables, on peut également citer les acyllactylates dont le groupe acyle comporte de 8 à 20 atomes de carbone.

En outre, on peut encore citer les acides d'alkyl-D-galactoside uroniques et leurs sels ainsi que les acides alkyl ($C_6$-$C_{24}$)éther-carboxyliques polyoxyalkylénés, les acides alkyl($C_6$-$C_{24}$)aryl($C_6$-$C_{24}$)éther-carboxyliques polyoxyalkylénés, les acides alkyl($C_6$-$C_{24}$)amidoéther carboxyliques polyoxyalkylénés et leurs sels, en particulier ceux comportant de 2 à 50 groupements oxyde d'éthylène, et leurs mélanges.

Parmi les tensioactifs anioniques cités ci-dessus, on préfère utiliser selon l'invention les alkyl($C_6$-$C_{24}$)sulfates, les alkyl($C_6$-$C_{24}$)éthersulfates, les alkyl($C_6$-$C_{24}$)éthercarboxylates et leurs mélanges, par exemple le laurylsulfate d'ammonium, le laurylsulfate de sodium, le laurylsulfate de magnésium, le lauryléthersulfate de sodium, le lauryléthersulfate d'ammonium et le lauryléthersulfate de magnésium.

Les agents tensioactifs non-ioniques que l'on peut utiliser dans le cadre de la présente invention, sont eux aussi, des composés bien connus en soi (voir notamment à cet égard "Handbook of Surfactants" par M.R. PORTER, éditions Blackie & Son (Glasgow and London), 1991, pp 116-178) Ils peuvent être notamment choisis parmi les alcools, les alpha-diols, les alkyl(C1-C20)phénols ou les acides gras polyéthoxylés, polypropoxylés ou polyglycérolés, ayant une chaîne grasse comportant par exemple de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et d'oxyde de propylène, les condensats d'oxyde d'éthylène et d'oxyde de propylène sur des alcools gras ; les amides gras polyéthoxylés ayant de préférence de 2 à 30 moles d'oxyde d'éthylène ; les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acide gras du polyéthylèneglycol, les alkyl($C_6$-$C_{24}$)Polyglucosides, les dérivés de N-alkyl($C_6$-$C_{24}$)glucamine, les oxydes d'amines tels que les oxydes d'alkyl($C_{10}$-$C_{14}$)amines ou les oxydes de N-acyl($C_{10}$-$C_{14}$)aminopropylmorpholine; et leurs mélanges.

Parmi les tensioactifs non-ioniques cités ci-dessus, on utilise de préférence les alkyl($C_6$-$C_{24}$)polyglycosides, en particulier le décylpolyglucoside.

La composition selon l'invention peut comprendre en outre un ou plusieurs tensioactifs cationiques bien connus en soi, tels que les sels d'amines grasses primaires, secondaires ou tertiaires, éventuellement polyoxyalkylénées, les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium, les dérivés d'imidazoline ; ou les oxydes d'amines à caractère cationique.

Les agents tensioactifs non ioniques, amphotères, anioniques et cationiques décrits ci-dessus peuvent être utilisés seuls ou en mélanges.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant 8 à 22 atomes de carbone et contenant, au moins un groupe anionique hydrosolubilisant tel que, par exemple, un groupe carboxylate, sulfonate, sulfate, phosphate ou phosphonate, on peut citer encore les alkyl($C_8$-$C_{20}$)bétaïnes, les sulfobétaïnes, les alkyl($C_8$-$C_{20}$)amidoalkyl($C_6$-$C_8$)-bétaïnes ou les alkyl($C_8$-$C_{20}$)amidoalkyl($C_6$-$C_8$)sulfobétaïnes ; et leurs mélanges.

Le ou les tensioactifs représentent de 0,01 à 60% en poids, de préférence de 0,1 à 30% et idéalement 1 à 20% en poids par rapport au poids total de la composition.

De manière tout à fait préférée, la composition selon l'invention comprend un tensioactif, choisi parmi un tensioactif non-ionique, cationique ou des mélanges de ceux-ci.

Le troisième constituant essentiel des compositions de l'invention est un corps gras.

Par corps gras, on entend, un composé organique insoluble dans l'eau à température ordinaire (25°C) et à pression atmosphérique (760 mm de Hg) (solubilité inférieure à 5% et de préférence à 1% encore plus préférentiellement à 0.1 %) et possédant des propriétés lubrifiantes. Notamment, au sens de la présente invention, un corps gras est un composé choisi parmi un alcool gras, un acide gras, un ester d'acide gras, un ester d'alcool gras, une huile minérale, végétale, animale ou synthétique, une silicone ou une cire.

Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité ;
- les hydrocarbures linéaires ou ramifiés, d'origine minérale ou synthétique, tels que les huiles de paraffine, volatiles ou non, et leurs dérivés, la vaseline, l'huile de vaseline, les polydécènes, le polyisobutène hydrogéné tel que Parléam® ; les isoparaffines comme l'isohexadécane et l'isodécane.
- les alcools gras sont saturés ou insaturés, linéaires ou ramifiés, et comportent de 8 à 30 atomes de carbone, on peut citer l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ; comme huiles fluorées, on peut citer aussi le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane ; les perfluoroalcanes tels que le dodécafluoropentane et

le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M ;

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les acides gras peuvent être saturés ou insaturés et comportent de 9 à 30 atomes de carbone. Ils sont choisis plus particulièrement parmi l'acide myristique, l'acide palmitique, l'acide stéarique, l'acide béhénique, l'acide oléïque, l'acide linoléïque, l'acide linolénique et l'acide isostéarique,

Les esters d'acides carboxyliques sont en particulier les esters mono, di, tri ou tétracarboxyliques.

Les esters sont les esters de mono ou polyacides aliphatiques saturés ou insaturés, linéaires ou ramifiés en $C_1$-$C_{26}$ et de mono ou polyalcools aliphatiques saturés ou insaturés, linéaires ou ramifiés en $C_1$-$C_{26}$ , le nombre total de carbone des esters étant supérieur ou égal à 10.

Parmi les monoesters , on peut citer le béhénate de dihydroabiétyle ; le béhénate d'octyldodécyle ; le béhénate d'isocétyle ; le lactate de cétyle ; le lactate d'alkyle en $C_{12}$-$C_{15}$ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le malate de dioctyle, le laurate d'hexyle, le laurate de 2-hexyldécyle.

On peut également utiliser les esters d'acides di ou tricarboxyliques en $C_4$-$C_{22}$ et d'alcools en $C_1$-$C_{22}$ et les esters d'acides mono di ou tricarboxyliques et d'alcools di, tri, tétra ou pentahydroxy en $C_2$-$C_{26}$.
On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

Les silicones utilisables en tant qu'additifs dans les compositions cosmétiques de la présente invention, sont des silicones volatiles ou non volatiles, cycliques, linéaires ou ramifiées, modifiées ou non par des groupements organiques, ayant une viscosité de $5.10^{-6}$ à $2{,}5m^2/s$ à 25°C et de préférence $1.10^{-5}$ à $1m^2/S$.

Les silicones utilisables conformément à l'invention peuvent être solubles ou insolubles dans la composition et en particulier être des polyorganosiloxanes insolubles dans la composition de l'invention. Elles peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:

(i) les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207

par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges.

On peut également citer les cyclocopolymères du type diméthylsiloxanes/ méthylalkylsiloxane, tel que la SILICONE VOLATILE® FZ 3109 commercialisée par la société UNION CARBIDE, de formule :

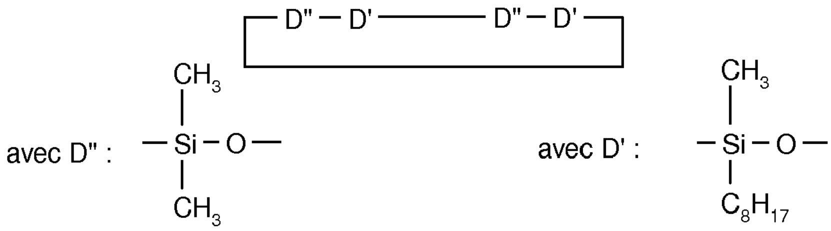
avec D" : avec D' :

On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;

(ii) les polydialkylsiloxanes volatiles linéaires ayant 2 à 9 atomes de silicium et présentant une viscosité inférieure ou égale à $5.10^{-6} m^2/s$ à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :

- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 $mm^2/s$ ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl ($C_1$-$C_{20}$) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :

- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile

PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6} m^2/s$. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs : $R_2SiO_{2/2}$, $R_3SiO_{1/2}$, $RSiO_{3/2}$ et $SiO_{4/2}$
dans lesquelles R représente un alkyl possédant 1 à 16 atomes de carbone. Parmi ces produits, ceux particulièrement préférés sont ceux dans lesquels R désigne un groupe alkyle inférieur en $C_1$-$C_4$, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkyl-arylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de $1.10^{-5}$ à $5.10^{-2} m^2/s$ à 25°C.

Parmi ces polyalkylarylsiloxanes on peut citer à titre d'exemple les produits commercialisés sous les dénominations suivantes :

. les huiles SILBIONE® de la série 70 641 de RHODIA;
. les huiles des séries RHODORSIL® 70 633 et 763 de RHODIA ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000 ;
certaines huiles des séries SF de GENERAL ELECTRIC telles que SF 1023, SF 1154, SF 1250, SF 1265.

Parmi les silicones organomodifiées, on peut citer les polyorganosiloxanes comportant :

- des groupements polyéthylèneoxy et/ou polypropylèneoxy comportant éventuellement des groupements alkyle en $C_6$-$C_{24}$ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl ($C_{12}$)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en $C_1$-$C_4$ ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

De préférence, l'acide stéarique est utilisé pour obtenir l'ester de polyéthylène glycol décrit ci-dessus.

De préférence, le corps gras est un alcool gras saturé ou un monoester d'acide gras ou de l'huile de vaseline.

Les corps gras représentent en général de 0,1 à 50%, de préférence 1 à 30%, et encore plus préférentiellement de 2 à 20% en poids de la composition totale.

Le milieu aqueux cosmétiquement acceptable peut contenir différents adjuvants et solvants couramment utilisés dans le domaine cosmétique tels que des agents gélifiants et/ou épaississants, des solvants organiques, des parfums, des pigments et colorants, des particules minérales ou organiques, des agents de stabilisation du pH, des agents conservateurs et des agents absorbant les UV.

Les agents gélifiants et/ou épaississants convenant pour les compositions de l'invention sont bien connus dans la technique et peuvent être choisis parmi les polymères et copolymères carboxy-vinyliques, les polymères et copolymères (alkyl)acryliques, les polymères et copolymères (alkyl)acrylamides, les poly(oxyalkylène)glycols, les esters de poly(oxyalkylène)glycols, les alginates, les biosaccharides, les polysaccharides tels que les dérivés de cellulose et d'amidon, les gommes naturelles telles que les gommes de xanthane, de guar, de caroube, les scléroglucanes, les dérivés de chitine et de chitosane, les carraghénanes, les argiles, et leur mélanges.

A titre d'exemple d'agents gélifiants, notamment en phase aqueuse, on peut citer le SEPIGEL® 305 commercialisé par la société SEPPIC, le FUCOGEL® 1000 PP commercialisé par la société SOLABIA, le SYNTHALEN® K commercialisé par la société 3VSA, le LUVISKOL® VA 64 P commercialisé par la société BASF, l'HOSTACERIN® AMPS commercialisé par la société CLARIANT, le PEMULEN® TR1 commercialisé par la société GOODRICH, le LUBRAGEL® MS commercialisé par la société GUARDIAN, le SATIAGEL® KSO commercialisé par DEGUSSA et le KELTROL® commercialisé par la société KELCO.

L'agent gélifiant représente en général de 0,1 à 15%, de préférence de 0,2 à 10% en poids de la composition.

Le milieu aqueux cosmétiquement acceptable de la composition peut, outre de l'eau, également contenir un ou plusieurs solvants organiques.

Le solvant organique est généralement choisi parmi les alcools $C_1$-$C_6$, de préférence les alcanols tels que l'éthanol, le propanol et l'isopropanol, les alcanediols tels que le propylène glycol et les pentanediols, l'acool benzylique, les alcanes en $C_5$-$C_{10}$, l'acétone, la méthyléthylcétone, l'acétate de méthyle, l'acétate de butyle, l'acétate d'éthyle, le diméthoxyéthane, le diéthoxyéthane et leurs mélanges.

En général, le solvant organique est présent à raison de 0,5 à 80% et de préférence de 1 à 50% du poids de la composition.

L'homme du métier saurait ajouter les additifs sans perturber les propriétés des compositions de l'invention.

Les compositions de l'invention sont de préférence des compositions de coiffage telles que des crèmes de coiffage. Bien entendu, elles peuvent être utilisées pour des produits coiffants rincés ou non-rincés.

Les compositions selon l'invention peuvent se présenter sous toutes les formes appropriées pour une application topique, notamment sous forme de solutions du type lotion ou sérum ; sous forme de gels aqueux ; sous forme d'émulsions obtenues par dispersion d'une phase grasse dans une phase aqueuse (H/E) ou inversement (E/H), de consistance liquide plus ou moins épaisse telle que des laits et des crèmes plus ou moins onctueuses ; sous forme de mousse, sous forme de spray, ou d'aérosol ; ou encore sous forme de stick.

Lorsque les compositions de l'invention sont conditionnées sous forme d'aérosol, elles comprennent un agent propulseur. L'agent propulseur, comme cela est bien connu, peut être un gaz ou mélange de gaz comprimé ou liquéfié, éventuellement dissous dans la composition. A titre d'exemple de gaz propulseur on peut citer, l'air, le gaz carbonique, l'azote, le diméthyléther, les hydrocarbures tels que le propane, le n-butane, l'isobutane ou l'isopentane et les hydrocarbures hologénés, en particulier fluorés Dans les compositions de l'invention, les silicones peuvent être initialement introduites dans la composition ou mélangés à la composition juste avant l'application.

## Exemples de formulation

**Exemple** 1 - **Crème de coiffage :**

| | |
|---|---|
| Polyuréthane cationique à séquence polyoléfine[1] | 2% m.a. |
| Alcool cétylstéarylique | 5% m.a |
| Mélange myristate/palmitate/stéarate de myristyle/cétyle/stéaryle | 1% m.a |
| GENAMIN KDMP[2] (commercialisé par CLARIANT) | 0,8% m.a |
| DC 939 EMULSION[3] (commercialisé par DOW CORNING) | 1,5% m.a. |
| Eau déminéralisée | Qsp 100% |
| [1] Polyuréthane (A) en dispersion aqueuse formé à partir de 8,7% de N-méthyl diéthanolamine, de 23,4% d'isophorone diisocyanate, de 67,9% de KRASOL LBH2000 (polybutadiène à fonctions terminales hydroxyles) et neutralisé à 40% par HCL.<br>[2]Chlorure de béhenyltrimonium<br>[3]Amodiméthicone | |

Appliquée sur les cheveux frisés, cette crème de coiffage assure une diminution de volume et une diminution du dessin de boucle durable.

**Exemple 2 - Emulsion :**

| | |
|---|---|
| Polyuréthane cationique à séquence polyoléfine[1] | 6% m.a. |
| Huile de vaseline MARCOL 82 | 36,7% m.a |
| Vaseline blanche CODEX 236 (Commercialisé par AIGLON) | 4% m.a |
| DOW CORNING 1664 Emulsion[2] (Commercialisé par DOW CORNING | 0,8% m.a |
| Glycérol | 4% m.a |
| TWEEN 20[3] (Commercialisé par UNIQEMA) | 2% m.a |
| Alcool cétylstéarylique | 2%m.a. |
| ARLACEL 165 FL[4] | 4,5% m.a. |
| Eau déminéralisée | Qsp 100% |
| [1] Polyuréthane (A) de l'exemple 1<br>[2] Polydiméthyl siloxane en émulsion aqueuse dans un mélange de lauryl éthers polyoxyéthylénés<br>[3] Monolaurate de sorbitane oxyéthyléné<br>[4] Mélange mono/distéarate de glycéryle/ stéarate de polyéthylèneglycol (100 OE) | |

Appliquée sur cheveux africains défrisés, cette émulsion permet d'obtenir des coiffures plaquées avec une longue tenue dans le temps (plusieurs jours). Appliquée sur cheveux très frisés type brésiliens, cette émulsion permet d'obtenir un très bon contrôle du volume et une définition de frisure avec une longue tenue dans le temps.

Un résultat similaire est obtenu en utilisant un polyuréthane (B) en dispersion aqueuse formé à partir de 8,4% de poly(tetraméthylène oxyde), 8,6% de N-méthyl diéthanolamine, de 21,4% d'isophorone diisocyanate, de 61,6% de KRATON L2203 polybutadiène à fonctions terminales hydroxyles) et neutralisé à 40% par HCL.

**Revendications**

**1.** Composition cosmétique comprenant, dans un milieu aqueux cosmétiquement acceptable :

(i) au moins un polyuréthane cationique comportant au moins un motif non ionique dérivé d'un homopolymère ou d'un copolymères d'oléfine, et
(ii) au moins un agent tensioactif, et
(iii) au moins un corps gras.

**2.** Composition cosmétique selon la revendication 1, **caractérisée en ce que** 50% en poids ou plus des motifs non ioniques du polyuréthane dérivent d'un homopolymère ou copolymère d'oléfine.

**3.** Composition cosmétique selon la revendication 1, **caractérisée en ce que** la totalité des motifs non ioniques du polyuréthane dérivent d'un homopolymère ou copolymère d'oléfine.

**4.** Composition cosmétique selon l'une quelconque des revendications 1 à 3, **caractérisée en ce** les homopolymères et copolymères d'oléfines sont des homopolymères et copolymères portant à leurs extrémités des fonctions à hydrogène labile, et présentant des motifs choisis parmi les motifs d'éthylène, de propylène, de 1-butylène, de 2-butylène, d'isobutylène, de 1,2-butadiène, de 1,4-butadiène, d'isoprène et leurs mélanges.

**5.** Composition cosmétique selon la revendication 4 **caractérisée en ce que** les homopolymères et copolymères d'oléfines sont issus de 1,2 et/ou 1,4 butadiène éventuellement hydrogénés.

**6.** Composition cosmétique selon l'une quelconque des revendications précédentes **caractérisée en ce que** le polyuréthane cationique comprend:

(a) des motifs cationiques résultant de la réaction d'au moins une amine tertiaire ou quaternaire présentant au moins deux fonctions réactives à hydrogène labile,
(b) des motifs non ioniques dont au moins un motif (b1) résulte de la réaction d'au moins un polymère choisi

parmi les homopolymères et les copolymères d'oléfines portant à leurs extrémités des fonctions réactives à hydrogène labile et ayant une température de transition vitreuse (Tg), mesurée par analyse enthalpique différentielle, inférieure à 10°C, et
(c) des motifs résultant de la réaction d'au moins un diisocyanate.

**7.** Composition cosmétique selon la revendication 6, **caractérisée en ce que** les motifs cationiques (a) résultent de la réaction d'au moins une amine tertiaire ou quaternaire présentant deux fonctions réactives à hydrogène labile.

**8.** Composition cosmétique selon la revendication 7, **caractérisée en ce que** l'amine est choisie parmi les amines de formules suivantes :

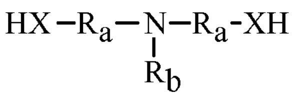
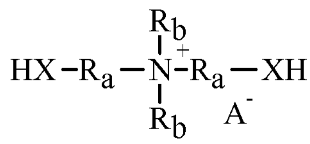

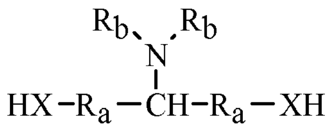
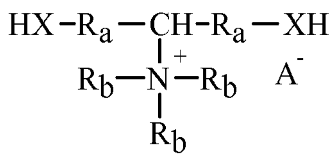

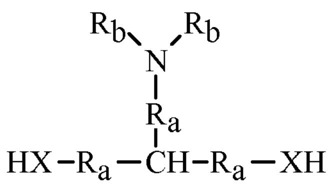
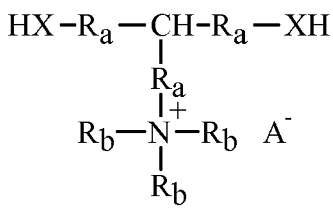

dans lesquelles
chaque $R_a$ représente indépendamment un groupe alkylène en $C_1$-$C_6$, linéaire ou ramifié, cycloalkylène en $C_3$-$C_6$ ou arylène, ou leurs mélanges ; tous pouvant être substitués par un ou plusieurs atomes d'halogène et comporter un ou plusieurs hétéroatomes choisis parmi O, N, P et S,
chaque $R_b$ représente indépendamment un groupe alkyle en $C_1$-$C_6$, cycloalkyle en $C_3$-$C_6$ ou aryle, ou leurs mélanges ; tous pouvant être substitués par un ou plusieurs atomes d'halogène et comporter un ou plusieurs atomes d'halogène et comporter un ou plusieurs hétéroatomes choisis parmi O, N, P et S,
chaque X représente indépendamment un atome d'oxygène ou de soufre ou un groupe NH ou $NR_c$, où $R_c$ représente un groupe alkyle en $C_1$-$C_6$, et
$A^-$ représente un contre-ion physiologiquement acceptable.

**9.** Composition cosmétique selon la revendication 8, **caractérisée en ce que** les motifs cationiques (a) résultent de la réaction de la N-méthyldiéthanolamine ou de la N-tert-butyldiéthanolamine.

**10.** Composition cosmétique selon la revendication 7, **caractérisée en ce que** les motifs (a) résultent de la réaction d'au moins un polymère à fonction(s) amine tertiaire et/ou quaternaire, portant à ses extrémités des fonctions réactives à hydrogène labile choisies parmi -OH, $-NH_2$, $-NHR_c$ ou -SH, et ayant une masse moléculaire en poids comprise entre 400 et 10 000, $R_c$ représentant un groupe alkyle en $C_1$-$C_6$.

11. Composition cosmétique selon l'une quelconque des revendications 6 à 10, **caractérisée en ce que** le polyuréthane cationique contient éventuellement au moins un motif non ionique (b2), différent du motif (b1), dérivant d'un composé non ionique monomère contenant au moins deux fonctions à hydrogène labile capables de réagir avec le ou lesdits motifs (c) contenant au moins un diisocyanate.

12. Composition cosmétique selon l'une quelconque des revendications 6 à 11, **caractérisée en ce que** les motifs cationiques (a) représentent de 0,1 à 90%, de préférence de 1 à 30%, mieux de 1 à 20% en poids, les motifs non-ioniques dérivés d'un homo- ou copolymère d'oléfine (b1) représentent de 10 à 99,9%, de préférence de 20 er141 à 99% et mieux de 30 à 85% en poids, et les motifs non-ioniques (b2) représentant de 0 à 50%, de préférence 0 à 30% en poids du total des motifs (a) et (b) du polyuréthane cationique.

13. Composition cosmétique selon l'une quelconque des revendications 6 à 11, **caractérisée en ce que** le diisocyanate est choisi parmi le méthylènediphényldiisocyanate, le méthylènecyclohexanediisocyanate, l'isophoronediisocyanate, le toluènediisocyanate, le naphtalènediisocyanate, le 1,4-butanediisocyanate et le 1,6-hexane-diisocyanate.

14. Composition cosmétique selon la revendication 13, **caractérisée en ce que** le diisocyanate est l'isophoronediisocyanate.

15. Composition cosmétique selon l'une quelconque des revendications 6 à 14, **caractérisée en ce que** les motifs (c) représentent de 1 à 60%, de préférence de 5 à 50%, et idéalement 1 à 40% du poids total des motifs du polyuréthane cationique.

16. Composition cosmétique selon l'une quelconque des revendications 6 à 15, **caractérisée en ce que** le ou les composés non ioniques monomères formant les motifs non ioniques (b2) sont choisis parmi les diols en $C_1$-$C_{12}$, de préférence le néopentylglycol, l'hexa(éthylèneglycol), le 1,2-éthanediol, le 1,2-propanediol et le 1,3-propanediol, et les aminoalcools en $C_1$-$C_6$, de préférence l'aminoéthanol.

17. Composition cosmétique selon l'une quelconque des revendications 6 à 16, **caractérisée en ce que** le polyuréthane cationique ne comprend pas de motifs en plus des motifs (a), (b) et (c).

18. Composition cosmétique selon l'une quelconque des revendications 6 à 17, **caractérisée en ce que** le polyuréthane cationique présente un caractère élastique.

19. Composition selon l'une quelconque des revendications précédentes, **caractérisée en ce que** le (ou les) polyuréthane(s) cationique(s) représente(nt) de 0,01% à 40%, de préférence 0,05 à 20% et idéalement de 0,1 à 10% en poids rapporté à la composition cosmétique finale.

20. Composition selon l'une quelconque des revendications précédentes, **caractérisée en ce que** le tensioactif est choisi parmi un tensioactif anionique, non ionique, cationique, amphotère et leurs mélanges.

21. Composition selon la revendication 20, **caractérisée en ce que** le tensioactif est cationique ou non ionique.

22. Composition selon l'une quelconque des revendications précédentes, **caractérisée en ce que** le ou les tensioactifs représente(nt) 0,01 à 20% en poids, de préférence 0,1 à 5% en poids, par rapport au poids total de la composition.

23. Composition selon l'une quelconque des revendications précédentes, **caractérisée en ce que** le corps gras est choisi parmi un alcool gras, un acide gras, un ester d'acide gras, un ester d'alcool gras, une huile minérale, végétale, animale et synthétique, une silicone ou une cire.

24. Composition selon la revendication 23, **caractérisée en ce que** le corps gras est un monoester d'acide gras ou une huile de vaseline.

25. Composition selon l'une quelconque des revendications précédentes **caractérisée en ce que** le (ou les) corps gras représente(nt) 0, 1 à 50% en poids, de préférence 0,1 à 30%, et mieux encore de 2 à 20% en poids de la composition.

26. Composition selon l'une quelconque des revendications précédentes **caractérisée en ce qu'**elle comprend un ou plusieurs additifs choisis parmi les agents gélifiants et/ou épaississants, les solvants organiques, les parfums, les agents de stabilisation de pH, les agents conservateurs et les agents absorbants les UV.

**27.** Composition selon l'une quelconque des revendications précédentes **caractérisée en ce qu'**elle se présente sous la forme d'une émulsion.

**28.** Composition selon l'une quelconque des revendications précédentes **caractérisée en ce qu'**elle contient un gaz propulseur et est conditionnée sous forme d'un aérosol.

**29.** Procédé de coiffage comprenant l'application d'une composition selon l'une quelconque des revendications 1 à 28 sur les cheveux, un rinçage éventuel des cheveux, puis la mise en forme et le séchage des cheveux.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2815350
- FR 2833960
- JP 2295912 A


### Littérature non-brevet citée dans la description


- **M.R. PORTER.** Handbook of Surfactants. Blackie & Son, 1991, 116-178
- **WALTER NOLL.** Chemistry and Technology of Silicones. Academie Press, 1968
- Volatile Silicone fluids for cosmetics. **TODD ; BYERS.** Cosmetics and Toiletries. Janvier 1976, vol. 91, 27-32